Europäisches Patentamt

European Patent Office

Office européen des brevets

(19)

(11) Veröffentlichungsnummer: **0 012 305**
**B1**

(12) # EUROPÄISCHE PATENTSCHRIFT

(45) Veröffentlichungstag der Patentschrift:
**22.12.82**

(21) Anmeldenummer: **79104819.2**

(22) Anmeldetag: **01.12.79**

(51) Int. Cl.³: **A 61 K 39/395**

(54) Mittel zur Therapie von Immunkomplexerkrankungen.

(30) Priorität: **11.12.78 DE 2853453**

(43) Veröffentlichungstag der Anmeldung:
**25.06.80 Patentblatt 80/13**

(45) Bekanntmachung des Hinweises auf die Patenterteilung:
**22.12.82 Patentblatt 82/51**

(84) Benannte Vertragsstaaten:
**AT BE CH DE FR GB IT LU NL SE**

(56) Entgegenhaltungen:
**EP-A-0 010 309**
**CH-A-392 780**
*DE-A-2 533 183*
*DE-A-2 658 334*
**US-A-3 466 368**

**K.O. Vorlaender: Praxis d. Immuol. Stuttgart 1976 S. 312–313 Microbiol. Abstracts, Bd. II, Sect. B, 1968, S. 232–233**

(73) Patentinhaber: **BEHRINGWERKE AKTIENGESELLSCHAFT, Postfach 1140, D-3550 Marburg/Lahn (DE)**

(72) Erfinder: **Sedlacek, Hans-Harald, Dr., Sonnenhang 3, D-3550 Marburg/Lahn (DE)**
Erfinder: **Seiler, Friedrich Robert, Dr., Oberer Eichweg 10, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

## Mittel zur Therapie von Immunkomplexerkrankungen

Immunkomplexe entstehen durch Bindung von Antigenen an Antikörper. Je nach Mengenverhältnis der Antigene zu den Antikörpern bilden sich Immunkomplexe unterschiedlicher Zusammensetzung, d.h. Immunkomplexe mit Antigenüberschuss, im Äquivalenzbereich oder mit Antikörperüberschuss.

Durch Bindung des Antikörpers an das Antigen wird das Fc-Teil des Antikörpers immunologisch verändert (aktiviert). Derartig immunologisch verändertes Fc ist in der Lage, Komplement zu aktivieren, Thrombozyten zu aggregieren und sich an Leukozyten zu binden. Durch die Bindung des Fc an Thrombozyten oder Leukozyten werden von diesen Zellen Mediatoren freigesetzt. Die Komplementaktivierung und die Freisetzung von Mediatoren aus den angeführten Zellen bewirken Entzündungsreaktionen, die unter Beteiligung des Gerinnungssystems mit Auftreten von Thrombosen ablaufen können. Derartige Entzündungen können extravasal oder intravasal wie beispielsweise in der Niere oder in Blutgefässen der Extremitäten auftreten. In der Niere führen sie zu den gefürchteten Glomerulonephritiden. Extravasal können sie beispielsweise in Gelenken zu rheumatoiden Arthritiden führen. Immunkomplexe können sich des weiteren mit dem Fc-Teil der beteiligten Antikörper an Lymphozyten binden und die Immunreaktion des Patienten und damit seine Abwehr hemmen. Diese verschiedenen, durch Immunkomplexe verursachten Erkrankungen werden als Immunkomplexerkrankungen zusammengefasst. Die Pathophysiologie und das Auftreten von Immunkomplexerkrankungen ist im Report Nr. 606 der WHO übersichtlich dargestellt.

Die derzeitigen Therapiemöglichkeiten von Immunkomplexerkrankungen sind beschränkt. Symptomatisch werden Substanzen gegeben, welche die Entzündungsreaktion vermindern (z. B. Corticoide), oder es werden Immunsuppressiva verabreicht, welche die Antikörperproduktion vermindern, so dass weniger Immunkomplexe entstehen können, oder es werden Immunkomplexe aus dem Blutkreislauf durch Plasmaphorese entfernt. Eine andere Möglichkeit der Therapie von Immunkomplexerkrankungen besteht in der Applikation von Antikörpern, die spezifisch gegen das im Immunkomplex enthaltene Antigen gerichtet sind. Eine derartige Therapie soll die Phagozytierbarkeit der Immunkomplexe und damit ihre Elimination erleichtern. Dies tritt aber nur ein, wenn das phagozytierende System noch aufnahmefähig ist, d.h. wenn es nicht erkrankt oder bereits übersättigt ist. Ein weiterer Nachteil besteht darin, dass in den meisten Fällen das die Erkrankung auslösende Antigen unbekannt ist oder dass spezifische Antikörper gegen das fragliche Antigen fehlen oder nur beschränkt verfügbar sind.

Es wurde nun überraschend gefunden, dass Fc-Reaktanten die Komplementaktivierung, die Thrombozytenaggregation und die Leukozytenbindung von Immunkomplexen verhindern können. Sie vermindern des weiteren die Nephrotoxizität von Immunkomplexen und sind somit zur Therapie von Immunkomplexerkrankungen verwendbar.

Fc-Reaktanten sind Komplementfaktoren wie C1q, C3b oder C3d oder Antikörper, welche mit dem immunologisch veränderten (aktivierten) Fc-Teil des im Immunkomplex enthaltenen Antikörpers direkt oder indirekt reagieren. Voraussetzung bei Verwendung von Antikörpern als Fc-Reaktanten ist jedoch, dass sie selbst kein immunologisch aktives oder aktivierbares Fc-Teil besitzen.

Eine Verwendung des Komplementfaktors C1q zur Reinigung von Immunglobulinen ist in der DE-A-2 533 183 beschrieben.

Gegenstand der Erfindung ist somit ein Mittel zur Therapie von Immunkomplexerkrankungen, enthaltend einen oder ein Gemisch von Fc-Reaktanten, welche direkt oder indirekt mit dem Fc-Teil des am Immunkomplex beteiligten Antikörpers reagieren, und welche selbst kein immunologisch aktives oder aktivierbares Fc-Teil besitzen, und einen üblichen pharmazeutischen Zusatz für die lokale oder parenterale Applikation.

Im Sinne der Erfindung sind derartige Fc-Reaktanten beispielsweise die Komplementfaktoren C1q, C3b oder C3d. Es können jedoch auch Antikörper sein, welche aufgrund ihrer immunologischen Spezifität direkt oder indirekt mit immunologisch verändertem (aktiviertem) Fc von Antikörpern in Immunkomplexen reagieren, welche selbst jedoch kein immunologisch oder aktivierbares Fc-Teil besitzen.

Antikörper ohne immunologisch aktives Fc-Teil im Sinne der Erfindung sind beispielsweise enzymatische Spaltprodukte von Immunglobulinen wie $F(ab)_2$ oder Fab-Fragmente, wie sie beispielsweise durch Behandlung von Immunglobulinen mit Pepsin (Porter: Biochem. J. 73, 119, 1959) herstellbar sind. Ähnliche, durch chemische Spaltung erhaltene Spaltprodukte sind in US-A-3 466 368 beschrieben.

Im Sinne der Erfindung sind jedoch auch Antikörper mit chemisch inaktiviertem Fc-Teil, beispielsweise solche mit Sulfitolysebehandlung (Schultze, Heremanns: Molecular Biology of Human Proteins, Elsevier Amsterdam 1966) (DE-A-2 658 334) verwendbar.

Chemisch abgebautes humanes Gammaglobulin ist in der schweizerischen Patentschrift 392 780 beschrieben.

Antikörper, welche aufgrund ihrer immunologischen Spezifität direkt mit immunologisch verändertem (aktiviertem) Fc von Antikörpern in Immunkomplexen reagieren, sind im Sinne der Erfindung beispielsweise Rheumafaktoren, wie sie bei verschiedenen Erkrankungen vorkommen, oder Antikörper, gewonnen durch Immunisierung von Tieren mit Immunglobulinen, welche das immunologisch veränderte (aktivierte) Fc enthalten.

In K.O. Vorlaender, Praxis der Immunologie, Stuttgart 1976, werden auf den Seiten 312 und 313 das Auftreten, die Natur und die Spezifität von Rheumafaktoren behandelt. Zur Spezifität wird ausgesagt, dass Rheumafaktoren mit Fc-Fragmenten jedoch weniger mit Fab reagieren.

Zur Immunisierung verwendete Immunglobulinpräparationen sind beispielsweise Antigen-Antikörper-Immunkomplexe, oder Immunglobulinaggregate oder Spaltprodukte von Immunglobulinen, welche noch immunologisch veränderte (aktive) Fc-Teile besitzen, wie das Fc-Teil, gewonnen nach enzymatischer Spaltung des Immunglobulins mit Papain (Porter: Biochem. J. 73, 119, 1959), Plasmin (Haupt, Heide: Klin. Wschr. 47, 270, 1969) oder Pepsin (Porter 1959; Nisonoff et al.: Arch. Biochem. Biophys, 89, 230, 1960). Auch in den Microbiol. Abstracts, Bd. III, Sect. B, 1968, S. 232–233 ist eine Möglichkeit zur Herstellung von Fc-Fragmenten beschrieben.

In der veröffentlichten Europäischen Patentanmeldung 10 309 werden Mittel zur Prophylaxe und Therapie allergischer Reaktionen beschrieben, die Immunglobuline der Klasse IgG, in denen durch eine immunologische Reaktion am Molekül die Fc-Region verändert und aktiviert ist, oder die Fc-Fragmente aus solchermassen veränderten Immunglobulin-Molekülen enthalten.

Im Sinne der Erfindung sind jedoch auch Antikörper verwendbar, welche aufgrund ihrer Spezifität indirekt mit immunologisch verändertem (aktiviertem) Fc von Antikörpern in Immunkomplexen reagieren, beispielsweise Antikörper, welche mit Komplementfaktoren reagieren, die sich an das Fc-Teil von Immunglobulinen im Immunkomplexen heften wie Antikörper gegen C1q, C3b oder C3d.

Zur Therapie sollte das erfindungsgemässe Präparat parenteral, beispielsweise i.m., i.v. oder s.c. appliziert werden. Es ist jedoch auch die lokale Applikation denkbar. Zur lokalen oder parenteralen Applikation ist das erfindungsgemässe Präparat in eine dem Fachmann bekannte Formulierung zu bringen. Die Dosis sollte 1 ng bis 1 g Protein pro kg Körpergewicht pro Applikation betragen.

Die Erfindung ist in nachstehenden Beispielen näher erläutert.

Beispiel 1

Die komplementaktivierende Wirksamkeit von Immunkomplexen unterschiedlicher Zusammensetzung mit und ohne Behandlung mit einem erfindungsgemässen Präparat wurde im Test nach Kabat und Majer (Experimental Immunochemistry, 2. Auflage, Thomas Springfield) bestimmt. Die Ergebnisse sind in Tabelle 1 aufgeführt.

Tabelle 1
Komplementaktivierende Wirksamkeit von Immunkomplexproben nach Behandlung mit einem erfindungsgemässen Mittel zur Therapie von Immunkomplexerkrankungen (in %)

| Behandlung der Immunkomplexe (0,5 ml) mit | Immunkomplexe mit Antigenüberschuss | Immunkomplexe im Äquivalenzpunkt | Immunkomplexe mit Antikörperüberschuss | Immunglobulin aggregate |
|---|---|---|---|---|
| Kontrolle behandelt mit physiol. Salzlösung (0,1 ml) | 100% | 100% | 100% | 100% |
| Kaninchen-Antikörper (F(ab)$_2$-Fragment gegen Papain-Fc (0,1 ml; 1%ig; 24 h, 4°C) | 75% | 44% | 41% | 90% |
| Rheumafaktor (F-(ab)$_2$-Fragment; 0,1 ml; 1%ig; 24 h, 4°C | 70% | 44% | 50% | 86% |
| C1q (0,1 ml; 0,2%ig, 3 h, 40°C) nachfolgend mit Kaninchen-Antikörper (F(ab)$_2$-Fragment) gegen C1q (0,1 ml; 1%ig, 24 h 4°C | 20% | 15% | 14% | 53% |
| C1q (0,1 ml, 0,2%ig, 3 h, 4°C) | 21% | 25% | 22% | 30% |

Sie zeigen deutlich, dass die Behandlung von Immunkomplexen unterschiedlicher Zusammensetzung (im Antigen- oder Antikörperüberschuss oder im Äquivalenzpunkt) oder auch von Immunglobulinaggregaten mit einem erfindungsgemässen Präparat zu einer deutlich verminderten Komplementaktivierung führt.

Beispiel 2

Die thrombozytenaggregierende Wirksamkeit von Immunkomplexen mit und ohne Behandlung mit einem erfindungsgemässen Präparat wurde im Test nach Born (Nature 194, 927, 1962) bestimmt. Die Ergebnisse sind in Tab. 2 aufgeführt.

## Tabelle 2

Thrombozytenaggregierende Wirksamkeit von Immunkomplexproben nach Behandlung mit einem erfindungsgemässen Mittel zur Therapie von Immunkomplexerkrankungen (in %)
Behandlung der Immunkomplexe mit: Thrombozytenaggregation
(Mengenverhältnisse wie Beispiel 1)

| | |
|---|---|
| Kontrolle (behandelt mit physiologischer Salzlösung) | 100% |
| Kaninchen-Antikörper-(F(ab)$_2$-Fragment) gegen Papain-Fc | 80% |
| Rheumafaktor (F(ab)$_2$-Fragment) | 80% |
| Clq, nachfolgend mit Kaninchen Kaninchen-Antikörper (F(ab)$_2$-Fragment) gegen Clq | 0% |
| Clq | 56% |

Sie zeigen deutlich eine Hemmung der Thrombozytenaggregation durch ein erfindungsgemässes Präparat.

## Beispiel 3

Die Bindung von Immunkomplexen mit oder ohne Behandlung mit einem erfindungsgemässen Präparat an Fc-Rezeptoren von Lymphozyten wurde in dem Rosettentest nach Seiler et al. (Z. f. Immunitätsforschung 155, 62, 1978) bestimmt. Hierzu wurden Immunkomplexe aus Humanerythrozyten und Antikörpern gegen Humanerythrozyten gemäss der Veröffentlichung von Seiler et al. (Behring Inst. Mitt. 52, 26, 1972) hergestellt und deren Bindung an Mäuseleukozyten (isoliert aus Milzzellen, Seiler et al. 1972) im sog. Rosettentest (Details siehe Seiler et al. 1972) untersucht.

Die Ergebnisse sind in Tab. 3 aufgeführt.

## Tabelle 3

Inhibition der Bindung von Immunkomplexen an Fc-Rezeptoren von Leukozyten durch ein erfindungsgemässes Präparat

| Behandlung der Immunkomplexe mit | Hemmung der Immunkomplexbindung |
|---|---|
| Kontrolle behandelt mit physiologischer Kochsalzlösung | 0 |
| Clq　0,01% | 17% |
| 　　　0,025% | 44% |
| 　　　0,05% | 56% |

Sie zeigen deutlich eine Hemmung der Bindung von Immunkomplexen durch ein erfindungsgemässes Präparat an Fc-Rezeptoren von Leukozyten.

## Beispiel 4

Mäusen wurden Immunkomplexe an drei aufeinanderfolgenden Tagen intravenös injiziert und zwei Stunden nach der letzten Injektion wurde ein erfindungsgemässes Präparat verabreicht. 24 Stunden später wurden die Tiere getötet und immunfluoreszenzhistologisch in den Glomeruli der Niere Immunkomplexe nachgewiesen und semiquantitativ über Ausverdünnen des zum Nachweis benutzten fluoreszeinmarkierten Antiserums bestimmt (methodische Details siehe Sedlacek et al. Zeitschrift f. Immunitätsforschung 155, 61, 1978). Die Ergebnisse sind in Tabelle 4 wiedergegeben.

## Tabelle 4

Hemmung der Ablagerung von Immunkomplexen in den Nieren von Mäusen durch ein erfindungsgemässes Präparat

| Nach Applikation von Immunkomplexen Behandlung mit: | Hemmung der Ablagerung von Immunkomplexen in der Niere |
|---|---|
| Kontrolle (phys. Kochsalzlösung) | 0 |
| Kaninchen-Antikörper (F(ab)$_2$-Fragmente gegen Papain-Fc) | 50 |
| Clq | 60 |

Die Ergebnisse zeigen deutlich eine Abnahme der Ablagerung von Immunkomplexen in der Niere nach Behandlung der Tiere mit einem erfindungsgemässen Präparat.

## Patentansprüche

1. Ein Mittel zur Therapie von Immunkomplexerkrankungen, enthaltend einen oder ein Gemisch von Fc-Reaktanten, welche direkt oder indirekt mit dem Fc-Teil des am Immunkomplex beteiligten Antikörpers reagieren und welche selbst kein immunologisch aktives oder aktivierbares Fc-Teil besitzen, und einen üblichen pharmazeutischen Zusätzen für die lokale oder parenterale Applikation.

2. Mittel nach Anspruch 1, dadurch gekennzeichnet, dass der Fc-Reaktant der Komplementfaktor C1q, C3b oder C3d ist.

3. Mittel nach Anspruch 1, dadurch gekennzeichnet, dass der Fc-Reaktant ein durch enzymatische Spaltung gewonnenes F(ab)$_2$- oder Fab-Fragment eines Antikörpers ist.

4. Mittel nach Anspruch 1, dadurch gekennzeichnet, dass der Fc-Reaktant ein Antikörper ist, dessen Fc-Teil chemisch verändert (inaktiviert) wurde.

5. Mittel nach Anspruch 1, 3 oder 4, dadurch gekennzeichnet, dass der Fc-Reaktant aus einem Rheumafaktor gewonnen wird.

6. Mittel nach Anspruch 1, 3 oder 4, dadurch gekennzeichnet, dass der Fc-Reaktant ein Antikörper ist und von Tieren stammt, welche mit einem Immunglobulin oder dessen Fragment mit immunologisch verändertem (aktivem) Fc-Teil immunisiert wurden.

7. Mittel nach Anspruch 1, 3 oder 4, dadurch gekennzeichnet, dass der Fc-Reaktant ein Antikörper gegen einen der Komplementfaktoren C1q, C3b oder C3d oder aus einem solchen Antikörper gewonnen ist.

**Claims**

1. Agent for the therapy of immunocomplex diseases, containing one Fc-reagent or a mixture of Fc-reagents, which react directly or indirectly with the Fc-part of the antibody contained in the immunocomplex and which do not possess themselves a Fc-part which is immunologically active or capable of being activated and a pharmaceutical carrier suitable for local or parenteral administration.

2. An agent as claimed in claim 1, wherein the Fc-reagent is the complement factor C1q, C3b or C3d.

3. An agent as claimed in claim 1, wherein the Fc-reagent is a F(ab)₂ or Fab-fragment of an antibody obtained by enzymatical fission.

4. An agent as claimed in claim 1, wherein the Fc-reagent is an antibody the Fc-part of which has been modified chemically (inactivated).

5. An agent as claimed in any one of claims 1, 3 and 4, wherein the Fc-reagent is a rheumatoid factor.

6. An agent is claimed in anyone of claims 1, 3 and 4, wherein the Fc-reagent is an antibody produced by animals that have been immunized with an immunoglobulin or a fragment thereof with an immunologically modified (active) Fc-part.

7. An agent as claimed in any one of claims 1, 3 and 4, wherein the Fc-reagent is an antibody against one of the complement factors C1q, C3b or C3d or is produced from such an antibody.

**Revendications**

1. Moyen thérapeutique pour les maladies dues aux immuncomplexes, contenant une ou plusieurs substances réagissant avec Fc, qui réagissent directement ou indirectement avec la fraction Fc de l'anticorps participant à l'immuncomplexe et qui ne possèdent pas elles-mêmes de fraction Fc immunologiquement active ou activable, et un additif pharmaceutique usuel pour l'application locale ou parentérale.

2. Moyen selon la revendication 1, caractérisé en ce que la substance réagissant avec Fc est le facteur du complément C1q, C3b ou C3d.

3. Moyen selon la revendication 1, caractérisé en ce que la substance réagissant avec Fc est un fragment F(ab)₂ ou Fab d'un anticorps, obtenu par scission enzymatique.

4. Moyen selon la revendication 1, caractérisé en ce que la substance réagissant avec Fc est un anticorps dont la fraction Fc a été modifiée (inactivée) par voie chimique.

5. Moyen selon l'une quelconque des revendications 1, 3 ou 4, caractérisé en ce que la substance réagissant avec Fc provient d'un facteur rhumatoïde.

6. Moyen selon l'une quelconque des revendications 1, 3 ou 4, caractérisé en ce que la substance réagissant avec Fc est un anticorps et est extraite d'animaux qui ont été immunisés avec une immunoglobuline ou son fragment avec une fraction Fc immunologiquement modifiée (active).

7. Moyen selon l'une quelconque des revendications 1, 3 ou 4, caractérisé en ce que la substance réagissant avec Fc est un anticorps contre un des facteurs du complément C1q, C3b, C3d ou est obtenue à partir d'un tel anticorps.